# EUROPÄISCHE PATENTSCHRIFT

(11) **EP 2 868 311 B1**
(45) Veröffentlichungstag und Bekanntmachung des Hinweises auf die Patenterteilung: **31.05.2017**
(21) Anmeldenummer: 14158230.4
(22) Anmeldetag: 07.03.2014
(51) Int. Cl.: A61K 8/34, A61K 8/46, A61Q 11/00, A61K 8/81

(54) **Mund- und Zahnpflege- und Reinigungsmittel zur Verringerung der Wiederanfärbung von Zähnen**
Oral and tooth care and cleaning agents for reducing the subsequent backstaining of teeth
Produit de nettoyage buccal et de soin dentaire destiné à réduire la recoloration des dents

(30) Priorität: 30.10.2013 DE 102013222120
(43) Veröffentlichungstag der Anmeldung: 06.05.2015
(73) Patentinhaber: Henkel AG & Co. KGaA, 40589 Düsseldorf (DE)
(72) Erfinder: Miehlich, Kristin, 42119 Wuppertal (DE); Adelmann, Barbara, 40597 Düsseldorf (DE); Arians, Daniela, 45239 Essen (DE); Hundeiker, Claudia, 40667 Meerbusch (DE); Eckert, Angelina, 47608 Geldern (DE)

(56) Entgegenhaltungen:
- JP-A- H0 314 512
- JP-A- 2005 187 377
- US-A1- 2003 157 033
- US-A1- 2006 140 884
- US-B1- 6 315 987

## Beschreibung

Die Erfindung betrifft Mund- und Zahnpflegemittel mit einer Wirkstoffkombination zur schonenden und effektiven Reinigung der Zähne.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Viele Menschen wünschen sich weiße Zähne und empfinden dunkle oder verfärbte Zähne als kosmetisch inakzeptabel. Die Aufrechterhaltung der natürlichen Zahnfarbe ist jedoch trotz regelmäßiger Dentalhygiene nicht immer erfolgreich. Ernährungsgewohnheiten oder Rauchen können zu Zahnverfärbungen führen. Ebenso führt die Besiedlung der Zahnoberfläche mit Bakterien (Plaque) zu Verfärbungen.

Eine Reihe von technischen Lösungen zur Entfernung oder Aufhellung von Zähnen wurde entwickelt. Zur Aufhellung/Bleichen kommt vor allem Peroxid zum Einsatz. In professionellen Bleichprodukten wird Peroxid in hohen Konzentrationen eingesetzt, während der Einsatz in Kosmetikprodukten zur Mund- und Zahnpflege auf 0,1% Peroxid beschränkt ist. Peroxid in dieser Konzentration hat nur eine eingeschränkte aufhellende Wirkung und kann Verfärbungen der Zähne oft nicht im gewünschten Maß beseitigen.

Eine weitere Möglichkeit zur Aufhellung der Zähne besteht in der effektiven Entfernung von Zahnbelägen, welche die Zähne dunkler aussehen lassen. Diese Methode der Zahnaufhellung wird auch als "Natural Whitening" beschrieben. Eine hohe Reinigungsleistung wird am besten erreicht durch Putzkörper, zum Beispiel Silica, Alumina oder Calciumcarbonat in Kombination mit einem Tensid. Leider weisen Zahncremes mit einem effektiven System aus einem oder mehreren Putzkörpern häufig auch eine hohe Abrasivität auf, führen also zu einem gewissen, wenn auch sehr geringem Abrieb der Zahnoberfläche. Dies kann sich insbesondere dann nachteilig auswirken, wenn das Enamel eines Zahnes ohnehin dünn ist, wie dies bei Personen mit empfindlichen Zähnen der Fall ist. Oft treten bei Personen mit empfindlichen Zähnen auch freiliegende Zahnhälse auf, also Partien des Zahnes in unmittelbarer Nähe zum Zahnfleisch, an denen kein Enamel als Schutzschicht vorhanden ist und das darunter liegende Dentin frei liegt.

Zudem bildet sich sofort nach dem Zähneputzen auf den Zahnmaterial eine Eiweißschicht (Pellicle) auf der sich der Plaque aufbaut bzw. Verfärbungen anlagern.

Es besteht daher ein Bedarf an Zahnpasten, die eine effektive Reinigung und Aufhellung bewirken aber dabei gleichzeitig einen Langzeiteffekt bewirken, d.h. die Bildung neuer Plaque und damit neuer Verfärbungen möglichst lange verhindern bzw. verringern. Gleichzeitig sollte eine möglichst lange antibakterielle Wirkung erzielt werden.

Plaquereduzierende und alkoholfreie Mundspülungen, die Polymere, Tensid sowie antibakterieelle Substanzen enthalten, sind aus der WO2012/018519 A1 bekannt. In ihrem Reinigungseffekt reichen Spüllösungen aber nicht an die mechanisch unterstützte Reinigung mittels Zahnbürste heran.

Der Einsatz von Isopropyl-Methylphenolen als antibakterielle Verbindungen wird beispielsweise in der WO 2013/083583 A1 offenbart. In dieser Schrift wird auch ein Synergismus zwischen diesen Verbindungen und Terpineolen gezeigt.

Die WO 2010/112578 A1 offenbart 4-Isopropyl-3-Methylphenol als entzündungshemmende Substanz sowie Mund- und Zahnpflege- und -reinigungsmittel, die 4-Isopropyl-3-Methylphenol enthalten.

Die JP 2005/187377 A offenbart in Beispiel 14 Zusammensetzungen, die Copolymere von Methylvinylether und dem Ethyl-Halbester der Maleinsäure sowie 3-Methyl-4-Isopropylphenol (p-Thymol) und Aniontensid enthalten.

Der Einsatz von Copolymeren aus Maleinsäure und Methylvinylether und Oregano-Extrakt bzw. Thymol wird in den Dokumenten US2006/140884 A1 und US2003/157033 A1 offenbart.

Die Aufgabe der vorliegenden Erfindung bestand darin, Zubereitungen zur Mund- und Zahnpflege und -reinigung bereitzustellen, die eine effektive Reinigung, Plaquereduktion und Aufhellung bewirken und dabei neue Verfärbungen möglichst lange verhindern bzw. verringern. Auch die Antibakterielle Wirkung der erfindungsgemäßen Mittel sollte länger anhaltend sein als die bekannter Mittel.

Überraschenderweise wurde nun gefunden, dass eine Kombination eines Polymers mit bestimmten Aktivstoffen dazu führt, dass Anfärbungen (z.B. aus der Nahrung, insbesondere Teeanfärbungen) sichtbar weniger aufgenommen werden. Die schonende Reinigung und hochwirksame Plaquereduktion wird durch eine solche Kombination um eine Langzeitwirkung gegen Wiederanschmutzung ergänzt.

Gegenstand der Erfindung sind daher Mund- und Zahnpflege- und -reinigungsmittel, enthaltend - bezogen auf sein Gewicht -
a) 0,001 bis 5 Gew.-% Copolymer(e), umfassend mindestens eine Struktureinheit der Formel (A1) und mindestens eine Struktureinheit der Formel (A2) 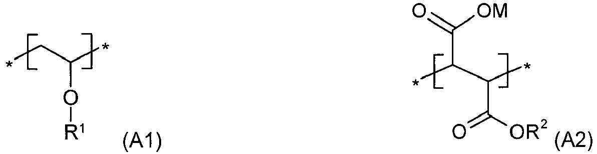 worin
   - R¹: steht für eine (C₁ bis C₁₈)-Alkylgruppe,
   - R²: steht für eine (C₁ bis C₆)-Alkylgruppe,
   - M: steht für ein Wasserstoffatom oder ein Äquivalent eines ein- oder mehrwertigen Kations,
b) 0,001 bis 5 Gew.-% mindestens eines Isopropylmethylphenols;
c) 0,1 bis 5 Gew.-% Aniontensid(e);
mit der Maßgabe, dass die Zusammensetzung 0,002 - 4 Gew.-% Thymol enthält.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Zusammensetzungen bezogen auf ihr Gewicht 0,001 bis 5 Gew.-% Copolymer(e), umfassend mindestens eine Struktureinheit der Formel (A1) und mindestens eine Struktureinheit der Formel (A2) 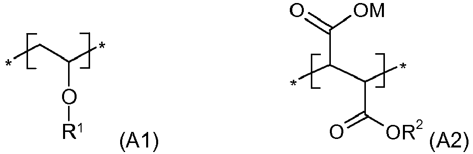 worin
- R¹: steht für eine (C₁ bis C₁₈)-Alkylgruppe,
- R²: steht für eine (C₁ bis C₆)-Alkylgruppe,
- M: steht für ein Wasserstoffatom oder ein Äquivalent eines ein- oder mehrwertigen Kations.

Gemäß obiger Formeln und allen folgenden Formeln steht eine chemische Bindung, die mit dem Symbol * gekennzeichnet ist, für eine freie Valenz des entsprechenden Strukturfragments.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl.

Beispiele für erfindungsgemäße (C₈ bis C₃₀)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Es ist erfindungsgemäß bevorzugt, wenn die erfindungsgemäßen Mittel als Komponete (b) mindestens ein Polymer enthalten, ausgewählt aus mindestens einem Polymer der Gruppe mit Polymeren der INCI-Nomenklatur Butyl Ester of PVM/MA Copolymer, Isopropyl Ester of PVM/MA Copolymer, Ethyl Ester of PVM/MA Copolymer.

Entsprechende Polymere der Komponete a) des erfindungsgemäßen Mittels werden beispielsweise unter dem Handelsnamen Gantrez^{®} ES 425 (Copolymer von Methylvinylether und dem Butyl-Halbester der Maleinsäure; 50 Gew.-% Aktivsubstanz in Ethanol; INCI-Bezeichnung: Butyl Ester of PVM/MA-Copolymer (Ashland)), Gantrez^{®} ES 435 (Copolymer von Methylvinylether und dem Butyl-Halbester der Maleinsäure; 50 Gew.-% Aktivsubstanz in Isopropanol; INCI-Bezeichnung: Butyl Ester of PVM/MA-Copolymer (Ashland)), Gantrez^{®} ES 335I (Copolymer von Methylvinylether und dem Isopropyl-Halbester der Maleinsäure; 50 Gew.-% Aktivsubstanz in Isopropanol; INCI-Bezeichnung: Butyl Ester of PVM/MA-Copolymer (Ashland)), Gantrez^{®} ES 225 (Copolymer von Methylvinylether und dem Ethyl-Halbester der Maleinsäure; 50 Gew.-% Aktivsubstanz in Ethanol) INCI-Bezeichnung: Ethyl Ester of PVM/MA-Copolymer (Ashland)).

Besonders bevorzugte erfindungsgemäße Mund- und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Copolymer(e) aus Maleinsäure und Methylvinylether enthalten.

Es hat sich gezeigt, dass Copolymere a) eines bestimmten Molmassenbereiches in der erfindungsgemäßen Kombination besonders wirksam sind. Besonders bevorzugte erfindungsgemäße Mund- und Zahnpflege- und reinigungsmittel sind daher dadurch gekennzeichnet, dass das/die Copolymer(e), umfassend mindestens eine Struktureinheit der Formel (A1) und mindestens eine Struktureinheit der Formel (A2), Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

Als zweiten wesentlichen Inhaltsstoff enthalten die erfindungsgemäßen Mittel 0,002 - 4 Gew.-% Thymol.

Thymol ist ein Monoterpen und neben seinem Isomer Carvacrol ein Bestandteil der ätherischen Öle aus Ajowan, Thymian, Oregano und dem Bohnenkraut. Thymol zeichnet sich durch eine starke desinfizierende fungizide und bakterizide Wirkung aus und wird wegen seines angenehmen Geschmacks in Mundwässern und Zahnpasta eingesetzt.

Bevorzugte erfindungsgemäße Mund- und Zahnpflege- und -reinigungsmittel sind dadurch gekennzeichnet, dass sie - bezogen auf ihr Gewicht 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Thymol enthalten.

Als dritten wesentlichen Inhaltsstoff enthalten die erfindungsgemäßen Zusammensetzungen bezogen auf ihr Gewicht 0,1 bis 5 Gew.-% Aniontensid(e).

Erfindungsgemäß bevorzugte Mund und Zahnpflege- und -reinigungsmittel sind dadurch gekennzeichnet, daß sie - bezogen auf ihr Gewicht - 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,25 bis 2,2 Gew.-% anionische(s) Tensid(e) enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono-und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte erfindungsgemäße Mittel enthalten Alkylsulfat(e) als anionisches Tensid. Hier sind erfindungsgemäß besonders bevorzugte Mund und Zahnpflege- und - reinigungsmittel sind dadurch gekennzeichnet, daß sie 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,25 bis 2,2 Gew.-% Natriumdodecylsulfat enthalten.

Die erfindungsgemäßen Mittel enthalten vorzugsweise einen oder mehrere Putzkörper, vorzugsweise aus der Gruppe der Fällungskieselsäuren. Vorzugsweise werden die Fällungskieselsäuren, die bevorzugt bestimmte spezifische Oberflächen aufweisen, innerhalb engerer Mengenbereiche eingesetzt, und insbesondere bevorzugt werden Fällungskieselsäuren eingesetzt, die niedrige spezifische Oberflächen nach ISO 5794-1, Anhang D aufweisen.

Bevorzugte erfindungsgemäße Mund- und Zahnpflege- und -reinigungsmittel enthalten 2,5 bis 19,5 Gew.-%, vorzugsweise 5 bis 19 Gew.-%, besonders bevorzugt 7,5 bis 18,5 Gew.-%, weiter bevorzugt 8,0 bis 18 Gew.-% und insbesondere 10,0 bis 17,5 Gew.-% Fällungskieselsäure(n) mit einer spezifischen Oberfläche nach ISO 5794-1, Anhang D von ≤ 60 m²/g, vorzugsweise von ≤ 52,5 m²/g, weiter bevorzugt von ≤ 49 m²/g und insbesondere von ≤ 47 m²/g.

In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Die erfindungsgemäßen Mittel können zusätzlich zu den genannten Fällungskieselsäuren a) weitere Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Aluminiumhydroxid, Aluminiumoxid, Natriumaluminiumsilikate, organische Polymere oder Gemische solcher Reibkörper.

Es ist bevorzugt, dass die erfindungsgemäßen Zusammensetzungen nur wenig bis keine Fällungskieselsäuren enthalten, die eine spezifische Oberfläche nach ISO 5794-1, Anhang D von > 55 m²/g aufweisen. Sollen solche Kieselsäuren eingesetzt werden, liegt das Gewichtsverhältnis von Fällungskieselsäuren mit einer spezifischen Oberfläche nach ISO 5794-1, Anhang D von ≤ 55 m²/g (Inhaltsstoff c)) zu Fällungskieselsäuren mit einer spezifischen Oberfläche nach ISO 5794-1, Anhang D von > 55 m²/g vorzugsweise > 1:1, weiter bevorzugt > 2:1, noch weiter bevorzugt > 5:1, besonders bevorzugt > 10:1 und insbesondere > 50:1.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Es hat sich gezeigt, daß die antibakterielle Wirkung der erfindungsgemäßen Mittel weiter gesteigert werden kann, wenn die Mittel Silcylsäure und/oder ihre Ester und/oder ihre Salze enthalten. Bevorzugte erfindungsgemäße Mittel enthalten bezogen auf ihr Gewicht 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Substanz(en) aus der Gruppe Salicylsäure, Natriumsalicylat, Kaliumsalicylat, Methylsalicylat, Ethylsalicylat.

Ganz besonders bevorzugte Mund und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Natriumsalicylat enthalten.

Als weiteren Inhaltsstoff können die erfindungsgemäßen Mittel 0,001 bis 10,0 Gew.-% mindestens eines Calciumsalzes enthalten. Besonders bevorzugt werden Calciumsalz(e) innerhalb engerer Mengenbereiche eingesetzt, so dass bevorzugte Mund und Zahnpflege- und -reinigungsmittel 0,05 bis 7,5 Gew.-%, vorzugsweise 0,1bis 5 Gew.-%, weiter bevorzugt 0,15 bis 2,5 Gew.-% und insbesondere 0,2 bis 1,25 Gew.-% Calciumsalz(e) enthalten.

Es lassen sich erfindungsgemäß alle physiologisch verträglichen Calciumsalze einsetzen, bevorzugt ist der Einsatz von Calciumsalzen, die einen weiteren Nutzen im erfindungsgemäßen Mund und Zahnpflege- und -reinigungsmittel entfalten. Unter diesen Verbindungen ganz besonders bevorzugt sind Calciumhydrogenphosphat-Dihydratn und/oder Calcium-Glycerophosphat.

Calciumhydrogenphosphat-Dihydrat, CaHPO₄•2H₂O wird je nach Literaturstelle auch als "Bruschit" oder als Dicalciumphosphat-dihydrat bezeichnet. Erfindungsgemäß bevorzugt ist der Einsatz von CaHPO₄•2H₂O, das durch die CAS-Nr: 7789-77-7 beschrieben wird.

Erfindungsgemäße Mund und Zahnpflege- und -reinigungsmittel, die als Calciumsalz Calciumhydrogenphosphat-Dihydrat, CaHPO₄•2H₂O enthalten, weisen deutliche Abrasivitätsvorteile gegenüber anderen Mitteln auf, die schonende Reinigung sensitiver Zähne ist daher mit der erfindungsgemäßen Kombination von Polymilchsäure, definiertem Silikat und Calciumhydrogenphosphat-Dihydrat als Calciumsalz noch einmal besser.

Es hat sich gezeigt, dass Calciumhydrogenphosphat-Dihydrat, CaHPO₄•2H₂O, vorzugsweise innerhalb enger Mengenbereiche eingesetzt wird. Hier sind erfindungsgemäße Mund und Zahnpflege- und -reinigungsmittel bevorzugt, die 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat enthalten.

Zusätzlich zu Calciumhydrogenphosphat-Dihydrat oder an seiner Stelle können die erfindungsgemäßen Mittel mit besonderem Vorzug Calcium-Glycerophosphat, d.h. ein Calciumsalz mindestens einer Glycerophosphorsäure enthalten.

Die Glycerophosphorsäure ist eine zweibasige Säure, die in zwei isomeren Formen vorkommt, je nachdem, ob die Phosphorsäuregruppierung an eine terminale oder die mediale OH-Gruppe des Glycerins gebunden vorliegt. Die Form, bei der die Phosphorsäuregruppierung an eine terminale OH-Gruppe des Glycerins gebunden vorliegt, wird auch als alpha-Isomer, die Form, bei der die Phosphorsäuregruppierung an die mediale OH-Gruppe des Glycerins gebunden vorliegt, auch als beta-Isomer bezeichnet.

Das alpha-Isomer ist zusätzlich optisch aktiv und tritt in den zwei enantiomeren Formen sn-Glycerol-1-phosphorsäure 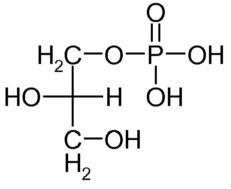 sowie sn-Glycerol-3-phosphorsäure 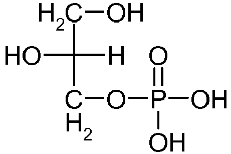 auf.

Das Präfix sn bei Glycerol-Derivaten steht für "stereospezifisch nummeriert" und verlangt, dass die 2-Hydroxy-Gruppe in der vorstehend verwendeten Fischer-Projektion nach links weist. Glycerol-2-phosphat ist nicht optisch aktiv. Die Glycerophosphorsäuren sind etwa so stark wie Phosphorsäure.

Erfindungsgemäß bevorzugt ist der Einsatz des alpha-Isomers, unabhängig davon, welches der beiden Enantiomere eingesetzt wird. Sofern der Einsatz enantiomerenreiner Verbindungen gewünscht ist, wird vorzugsweise das Calciumsalz der sn-Glycerol-3-phosphorsäure eingesetzt.

Zusammenfassend sind erfindungsgemäße Mund und Zahnpflege- und -reinigungsmittel bevorzugt, die Calciumsalze der Glycerophosphorsäuren der Formeln (la) und (Ib) enthalten HO-CH₂-CH(OH)-CH₂-OP(O)O₂²⁻ Ca²⁺ (Ia) HO-CH₂-CH(OP(O)O₂²⁻)-CH₂-OH Ca²⁺ (Ib).

Hierbei sind besonders bevorzugte erfindungsgemäße Mund und Zahnpflege- und reinigungsmittel dadurch gekennzeichnet, dass das Gewichtsverhältnis der Calciumsalze der Formeln (la) zu (Ib) oberhalb von 50:50, vorzugsweise oberhalb von 60:40, besonders bevorzugt oberhalb von 70:30 und insbesondere oberhalb 80:20 liegt.

Der Einsatz der Calcium-Glycerophosphate innerhalb engerer Mengenbereiche ist bevorzugt. Bevorzugte erfindungsgemäße Mund- und Zahnpflege- und -reinigungsmittel enthalten - bezogen auf ihr Gewicht - 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Ganz besonders bevorzugte erfindungsgemäße Mittel enthalten 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat und 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so dass Mittel, die außer Sorbit keine anderen mehrwertigen Alkohole enthalten, besonders bevorzugt sind.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Mund- und Zahnpflege- und reinigungsmittel können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Ganz besonders gut verträglich mit der erfindungsgemäßen Kombination ist Xanthan. Erfindungsgemäße Mittel, die Xanthan enthalten, sind außergewöhnlich lagerstabil und weisen eine angenehme Produkthaptik auf. Bevorzugte erfindungsgemäße Mund und Zahnpflege- und - reinigungsmittel sind daher dadurch gekennzeichnet, dass sie zusätzlich 0,1 bis 7,5 Gew.-%, vorzugsweise 0,25 bis 5 Gew.-%, weiter bevorzugt 0,5 bis 2,5 Gew.-% und insbesondere 0,6 bis 1,5 Gew.-% Xanthan enthalten.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Erfindungsgemäße Mittel können als Zahnpasten oder Zahncremes formuliert werden. Ein weiterer Gegenstand der vorliegenden Erfindung ist die Verwendung von erfindungsgemäßen Mitteln zur Reinigung von Zähnen mittels manuell betätigter oder elektrischer Zahnbürsten. Im Falle elektrischer Zahnbürsten besitzen die erfindungsgemäßen Mittel den weiteren Vorteil, dass sie bereits in geringen Mengen wirksam sind und darüber hinaus die Mechanik des elektrischen Bürstenkopfes nicht beeinträchtigen.

Durch den Einsatz der erfindungsgemäßen Mittel kann die Wiederanfärbung von Zähnen effektiv verringert werden.

## Patentansprüche

1. Mund- und Zahnpflege- und -reinigungsmittel, enthaltend - bezogen auf sein Gewicht -
a) 0,001 bis 5 Gew.-% Copolymer(e), umfassend mindestens eine Struktureinheit der Formel (A1) und mindestens eine Struktureinheit der Formel (A2) 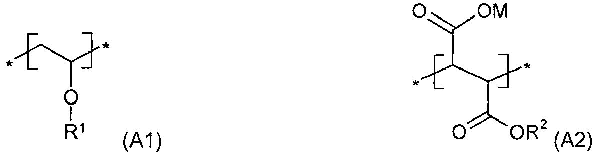 worin
R¹ steht für eine (C₁ bis C₁₈)-Alkylgruppe,
R² steht für eine (C₁ bis C₆)-Alkylgruppe,
M steht für ein Wasserstoffatom oder ein Äquivalent eines ein- oder mehrwertigen Kations;
b) 0,001 bis 5 Gew.-% mindestens eines Isopropylmethylphenols;
c) 0,1 bis 5 Gew.-% Aniontensid(e);
mit der Maßgabe, dass die Zusammensetzung 0,002 - 4 Gew.-% Thymol enthält.

2. Mund- und Zahnpflege- und -reinigungsmittel nach Anspruch 1, **dadurch gekennzeichnet, dass** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Copolymer(e) aus Maleinsäure und Methylvinylether enthält.

3. Mund- und Zahnpflege- und reinigungsmittel nach einem der Ansprüche 1 oder 2, **dadurch gekennzeichnet, dass** das/die Copolymer(e), umfassend mindestens eine Struktureinheit der Formel (A1) und mindestens eine Struktureinheit der Formel (A2), Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

4. Mund- und Zahnpflege- und -reinigungsmittel nach einem der Ansprüche 1 bis 3, **dadurch gekennzeichnet, dass** es - bezogen auf sein Gewicht - 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Thymol enthält.

5. Mund und Zahnpflege- und -reinigungsmittel nach einem der Ansprüche 1 bis 4, **dadurch gekennzeichnet, dass** es 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,25 bis 2,2 Gew.-% anionische(s) Tensid(e) enthält.

6. Mund und Zahnpflege- und -reinigungsmittel nach einem der Ansprüche 1 bis 5, **dadurch gekennzeichnet, dass** es 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,25 bis 2,2 Gew.-% Natriumdodecylsulfat enthält.

7. Mund und Zahnpflege- und -reinigungsmittel nach einem der Ansprüche 1 bis 6, **dadurch gekennzeichnet, dass** es 2,5 bis 19,5 Gew.-%, vorzugsweise 5 bis 19 Gew.-%, besonders bevorzugt 7,5 bis 18,5 Gew.-%, weiter bevorzugt 8,0 bis 18 Gew.-% und insbesondere 10,0 bis 17,5 Gew.-% Fällungskieselsäure(n) mit einer spezifischen Oberfläche nach ISO 5794-1, Anhang D von ≤ 60 m²/g, vorzugsweise von ≤ 52,5 m²/g, weiter bevorzugt von ≤ 49 m²/g und insbesondere von ≤ 47 m²/g enthält.

8. Mund und Zahnpflege- und -reinigungsmittel nach einem der Ansprüche 1 bis 7, **dadurch gekennzeichnet, dass** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-%, weiter bevorzugt 0,01 bis 0,5 Gew.-% und insbesondere 0,05 bis 0,2 Gew.-% Natriumsalicylat enthält.

9. Mund und Zahnpflege- und -reinigungsmittel nach einem der Ansprüche 1 bis 8, **dadurch gekennzeichnet, dass** es 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat und 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat enthält.

## Claims

1. An oral and dental care and cleaning agent, containing, based on the weight thereof
a) 0.001 to 5 wt.% copolymer(s), comprising at least one structural unit of formula (A1) and at least one structural unit of formula (A2) 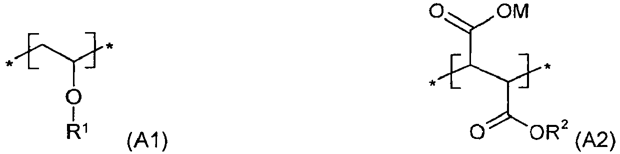 in which
R¹ represents a (C₁ to C₁₈) alkyl group,
R² represents a (C₁ to C₆) alkyl group,
M represents a hydrogen atom or an equivalent of a monovalent or polyvalent cation;
b) 0.001 to 5 wt.% of at least one isopropylmethylphenol;
c) 0.1 to 5 wt.% anionic surfactant(s);
provided that the composition contains 0.002 to 4 wt.% thymol.

2. The oral and dental care and cleaning agent according to claim 1, **characterized in that** it contains, based on the weight thereof, 0.002 to 4 wt.%, preferably 0.003 to 3 wt.%, particularly preferably 0.004 to 2 wt.%, exceptionally preferably 0.005 to 1 wt.% and in particular 0.01 to 0.5 wt.% copolymer(s) of maleic acid and methyl vinyl ether.

3. The oral and dental care and cleaning agent according to one of claims 1 or 2, **characterized in that** the copolymer(s), comprising at least one structural unit of formula (A1) and at least one structural unit of formula (A2), has/have a molar mass of from 500,000 to 5,000,000 daltons, preferably from 600,000 to 4,000,000 daltons, more preferably from 700,000 to 3,000,000 daltons, even more preferably from 800,000 to 2,000,000 daltons and in particular from 900,000 to 1,500,000 daltons.

4. The oral and dental care and cleaning agent according to one of claims 1 to 3, **characterized in that** it contains, based on the weight thereof, 0.003 to 3 wt.%, particularly preferably 0.004 to 2 wt.%, exceptionally preferably 0.005 to 1 wt.%, more preferably 0.01 to 0.5 wt.% and in particular 0.05 to 0.2 wt.% thymol.

5. The oral and dental care and cleaning agent according to one of claims 1 to 4, **characterized in that** it contains 0.25 to 4 wt.%, preferably 0.5 to 3.5 wt.%, more preferably 0.75 to 3 wt.%, even more preferably 1 to 2.5 wt.% and in particular 1.25 to 2.2 wt.% anionic surfactant(s).

6. The oral and dental care and cleaning agent according to one of claims 1 to 5, **characterized in that** it contains 0.25 to 4 wt.%, preferably 0.5 to 3.5 wt.%, more preferably 0.75 to 3 wt.%, even more preferably 1 to 2.5 wt.% and in particular 1.25 to 2.2 wt.% sodium dodecyl sulfate.

7. The oral and dental care and cleaning agent according to one of claims 1 to 6, **characterized in that** it contains 2.5 to 19.5 wt.%, preferably 5 to 19 wt.%, particularly preferably 7.5 to 18.5 wt.%, more preferably 8.0 to 18 wt.% and in particular 10.0 to 17.5 wt.% precipitated silica(s) having a specific surface area according to ISO 5794-1, Appendix D, of ≤ 60 m²/g, preferably ≤ 52.5 m²/g, more preferably ≤ 49 m²/g, and in particular ≤ 47 m²/g.

8. The oral and dental care and cleaning agent according to one of claims 1 to 7, **characterized in that** it contains, based on the weight thereof, 0.002 to 4 wt.%, preferably 0.003 to 3 wt.%, particularly preferably 0.004 to 2 wt.%, exceptionally preferably 0.005 to 1 wt.%, more preferably 0.01 to 0.5 wt.% and in particular 0.05 to 0.2 wt.% sodium salicylate.

9. The oral and dental care and cleaning agent according to one of claims 1 to 8, **characterized in that** it contains 0.25 to 7.5 wt.%, preferably 0.5 to 7.0 wt.%, particularly preferably 1.0 to 6.0 wt.%, more preferably 2.0 to 5.0 wt.% and in particular 3.5 to 4.5 wt.% calcium hydrogen phosphate dihydrate, and 0.01 to 2.5 wt.%, preferably 0.05 to 2.0 wt.%, particularly preferably 0.1 to 1.0 wt.%, more preferably 0.11 to 0.75 wt.% and in particular 0.12 to 0.5 wt.% calcium glycerophosphate.

## Revendications

1. Produit de nettoyage et de soin de la bouche et des dents, contenant - rapporté à son poids :
a) 0,001 à 5 % en poids d'au moins un copolymère, comprenant au moins une unité structurelle de formule (A1) et au moins une unité structurelle de formule (A2) : 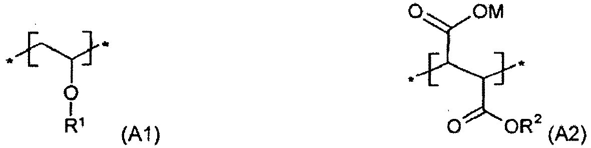 où
R¹ représente un groupe C₁₋₁₈-alkyle
R² représente un groupe C₁₋₆-alkyle
M représente un atome d'hydrogène ou un équivalent d'un cation mono ou polyvalent ;
b) 0,001 à 5 % en poids d'au moins un isopropylméthylphénol ;
c) 0,1 à 5 % en poids d'au moins un tensioactif ;
à condition que la composition contienne 0,002 à 4 % en poids de thymol.

2. Produit de nettoyage et de soin de la bouche et des dents selon la revendication 1, **caractérisé en ce qu'**il contient, rapporté à son poids, 0,002 à 4 %, de préférence 0,003 à 3 %, particulièrement préférentiellement 0,004 à 2 % en poids, extraordinairement préférentiellement 0,005 à 1 % en poids et en particulier 0,01 à 0,5 % en poids d'au moins un copolymère d'acide maléique et de méthylvinyléther.

3. Produit de nettoyage et de soin de la bouche et des dents selon l'une des revendications 1 ou 2, **caractérisé en ce que** l'au moins un copolymère comprenant au moins une unité structurelle de formule (A1) et au moins une unité structurelle de formule (A2) présente des masses molaires de 500 000 à 5 000 000 Dalton, de préférence 600 000 à 4 000 000 Dalton, plus préférentiellement 700 000 à 3 000 000 Dalton, encore plus préférentiellement 800 000 à 2 000 000 Dalton, et en particulier 900 000 à 1 500 000 Dalton.

4. Produit de nettoyage et de soin de la bouche et des dents selon l'une des revendications 1 à 3, **caractérisé en ce qu'**il contient, rapporté à son poids, 0,003 à 3 %, particulièrement préférentiellement 0,004 à 2 % en poids, extraordinairement préférentiellement 0,005 à 1 % en poids, encore plus préférentiellement 0,01 à 0,5 % en poids et en particulier 0,05 à 0,2 % en poids de thymol.

5. Produit de nettoyage et de soin de la bouche et des dents selon l'une des revendications 1 à 4, **caractérisé en ce qu'**il contient, rapporté à son poids, 0,25 à 4 %, de préférence 0,5 à 3,5 % en poids, plus préférentiellement 0,75 à 3 % en poids, encore plus préférentiellement 1 à 2,5 % en poids et en particulier 1,25 à 2,2 % en poids d'au moins un tensioactif anionique.

6. Produit de nettoyage et de soin de la bouche et des dents selon l'une des revendications 1 à 5, **caractérisé en ce qu'**il contient, rapporté à son poids, 0,25 à 4 %, de préférence 0,5 à 3,5 % en poids, plus préférentiellement 0,75 à 3 % en poids, encore plus préférentiellement 1 à 2,5 % en poids et en particulier 1,25 à 2,2 % en poids de dodécylsulfate de sodium.

7. Produit de nettoyage et de soin de la bouche et des dents selon l'une des revendications 1 à 6, **caractérisé en ce qu'**il contient, rapporté à son poids, 2,5 à 19,5 %, de préférence 5 à 19 % en poids, particulièrement préférentiellement 7,5 à 18,5 % en poids, plus préférentiellement 8,0 à 18 % en poids, et en particulier 10,0 à 17,5 % en poids d'au moins un acide silicique précipité avec une surface spécifique selon la norme ISO 5794-1 annexe D ≤ 60 m²/g, de préférence ≤ 52,5 m²/g, plus préférentiellement ≤ 49 m²/g et en particulier ≤ 47 m²/g.

8. Produit de nettoyage et de soin de la bouche et des dents selon l'une des revendications 1 à 7, **caractérisé en ce qu'**il contient, rapporté à son poids, 0,002 à 4 %, de préférence 0,003 à 3 % en poids, particulièrement préférentiellement 0,004 à 2 % en poids, extraordinairement préférentiellement 0,005 à 1 % en poids, plus préférentiellement 0,01 à 0,5 % en poids et en particulier 0,05 à 0,2 % en poids de salicylate de sodium.

9. Produit de nettoyage et de soin de la bouche et des dents selon l'une des revendications 1 à 8, **caractérisé en ce qu'**il contient, rapporté à son poids, 0,25 à 7,5 %, de préférence 0,5 à 7,0 % en poids, particulièrement préférentiellement 1,0 à 6,0 % en poids, plus préférentiellement 2,0 à 5,0 % en poids et en particulier 3,5 à 4,5 % en poids d'hydrogénophosphate de calcium dihydraté et 0,01 à 2,5 %, de préférence 0,05 à 2,0 % en poids, particulièrement préférentiellement 0,1 à 1,0 % en poids, plus préférentiellement 0,11 à 0,75 % en poids et en particulier 0,12 à 0,5 % en poids de glycérophosphate de calcium.
